# EUROPEAN PATENT SPECIFICATION

(11) **EP 0 368 568 B1**
(45) Date of publication and mention of the grant of the patent: **27.12.1996**
(21) Application number: 89311373.8
(22) Date of filing: 02.11.1989
(51) Int. Cl.: A61N 1/05

(54) **Apparatus for removing an elongated structure implanted in biological tissue**
Apparat zur Entfernung eines in einem biologischen Gewebe implantierten länglichen Elementes
Appareil pour retirer un élément longiforme implanté dans un tissu biologique

(30) Priority: 09.11.1988 US 269771; 17.01.1989 US 298100; 03.05.1989 US 347217; 09.06.1989 US 363960
(43) Date of publication of application: 16.05.1990
(60) Divisional application: 96109223.6
(73) Proprietor: MED INSTITUTE, INC., West Lafayette Indiana 47906 (US); COOK PACEMAKER CORPORATION, Leechburg Pennsylvania 15656 (US)
(72) Inventor: Goode, Louis, Evans City Pennsylvania 16033 (US); Shipko, Frederick John, Spring Church Pennsylvania 15686 (US); Fearnot, Neal Edward, West Lafayette Indiana 47906 (US)
(74) Representative: Johnston, Kenneth Graham

(56) References cited:
- US-A- 4 471 777
- US-A- 4 574 800

## Description

This invention relates to elongated structures, such as a catheter implanted in biological tissue, ducts etc. or an electrical pacemaker lead implanted in the heart and, particularly, to apparatus for removing such elongated structures.

A heart pacemaker comprising an electrical wire coil or lead usually is surgically implanted through a vein such as the right subclavian vein leading to a cavity such as the superior vena cava, and to the right ventricle of the heart. A typical lead includes one or more helical wire coils having a hollow inner passageway that extends the entire length of the wire coil. The coiled structures are positioned in the lead either coaxially or laterally, and are insulated from body fluids by insulating material (silicon or polyurethane). However, one problem is that, over the years, fibrotic tissue commonly encapsulates the pacemaker lead especially in areas where there is low velocity blood flow. Separation of the lead from the vein can cause severe damage to the vein.

Should a heart lead need to be replaced at any time, it is undesirable to leave the existing pacemaker lead in place.

If removal of a lead from the heart has been required for any reason, it has often involved open heart surgery with accompanying complications, risks, and significant cost.

Prior art apparatus or tools for removing pacemaker leads have not proved satisfactory. Once locked in position they cannot be disengaged from the locking relationship with the pacemaker leads. As a result, the tool and lead must be surgically removed. Furthermore, certain tools can easily puncture a blood vessel or, even worse, a heart cavity wall. It has been known for a coiled structure to unwind and damage the heart and surrounding blood vessels. Pacemaker leads can include tines or a corkscrew at the tip or a conically shaped tip for securing the distal end of the pacemaker lead to a heart cavity wall. For fibrotic tissue that has encapsulated the tip, unaided manual removal of the heart lead from the heart cavity wall may cause an inward extension or inversion of the wall, or even worse, permanent damage to the heart such as tearing a hole in the heart cavity wall.

U.S. Patent 4,574,800 discloses apparatus for removing implanted cardiac pace making leads, said apparatus being as defined in the preamble of claim 1.

U.S. Patent 4,471,777 also discloses apparatus for removing similar types of leads. The apparatus uses grasping claws for securing the proximal ends of such claws.

The present invention provides apparatus as defined in claim 1 for removing an elongated structure implanted in biological tissue.

### Brief Description of the Drawing

FIGS. 1,2 and 3 illustrate sections through apparatus useful for providing an understanding of the invention; they are not embodiments of this invention;
FIG. 4 depicts a device for expanding the proximal end of the coiled structure of FIG. 1 in order to facilitate insertion of the lead removal apparatus;
FIG. 5 depicts an embodiment of the present invention; and
FIG. 6 depicts an alternative embodiment of the present invention.

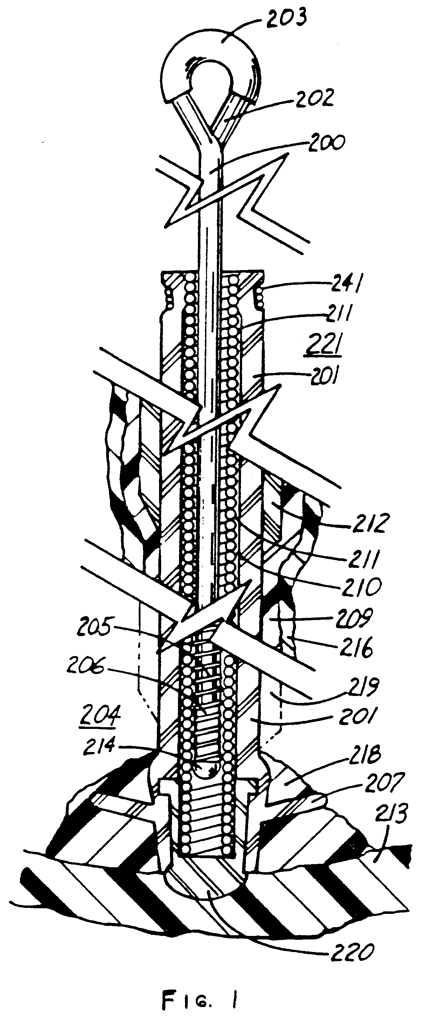

FIG. 1 shows an electrical heart pacemaker lead 204 secured to the apex 213 of the right ventricle of the heart with a plurality of tines 207, which in time become securely attached to the ventricle wall by endothelial and/or fibrotic tissue forming around the heart lead tip with electrode 220 in contact with the lead and the ventricle wall 213. Some ventricles are relatively smooth on the inside, but most have trabeculae amongst which the tines are secured into position. External to the right subclavian vein, the proximal end 221 of the lead can be grapsed by a lockable clamp mechanism.

A flexible stylet member such as wire 200 is inserted in the longitudinal passageway 210 of the heart lead coiled structure 211 for controlling and, in particular, limiting the movement of heart lead 204 including coiled structure 211. Heart lead 204 can also include insulating material 201, such as silicone or polyurethane, formed into a hollow tube that surrounds the coiled structure and prevents fluids from making contact with or entering the coiled structure. Attached to the flexible stylet wire is an outwardly expandable part 205 consisting of approximately 25 turns of wire with spacing between the turns. Five to seven wraps of the wire coil are attached preferably close to the distal end of the stylet wire using, for example, solder 206. The remaining wraps of the wire coil remain free for engaging the coiled structure when the proximal end of the stylet wire is rotated in a direction to unwind and expand the turns of the wire coil and engage the coiled structure of the heart lead. A bead 214 of high temperature silver solder is applied to the distal end of the stylet wire to prevent the distal end thereof from pulling through the wire coil during separation and removal of the heart lead. Positioned about the proximal end of the stylet wire is control mechanism 202 for rotating the stylet wire in either a clockwise or counterclockwise direction or for moving the wire in a longitudinal direction into or out of the passageway. The control mechanism 202 is a loop of wire formed from the stylet wire of which the physician may grasp or insert his finger.

The use of stainless steel stylet wire is preferable. The stylet wire should be hardened wire, but ductable wire may be used for the coil wire.

Before the stylet wire is inserted into passageway 210 of the lead, the inside diameter of the coiled structure and the outside diameter of the insulating material are determined. A lockable clamp is first applied to the proximal end 221 of the lead, the latter being in channels in pliable opposing semicircular jaws of the clamp.

When proximal end 221 of lead 204 is inserted and grasped in the hollow passageway, the insulating material 201 is compressed onto coiled structure 211, thus limiting the movement of the structure within the insulating material. When the physician cuts the lead for access to the passageway of the lead, the compressed insulating material prevents the coiled structure from retracting into the passageway of the lead.

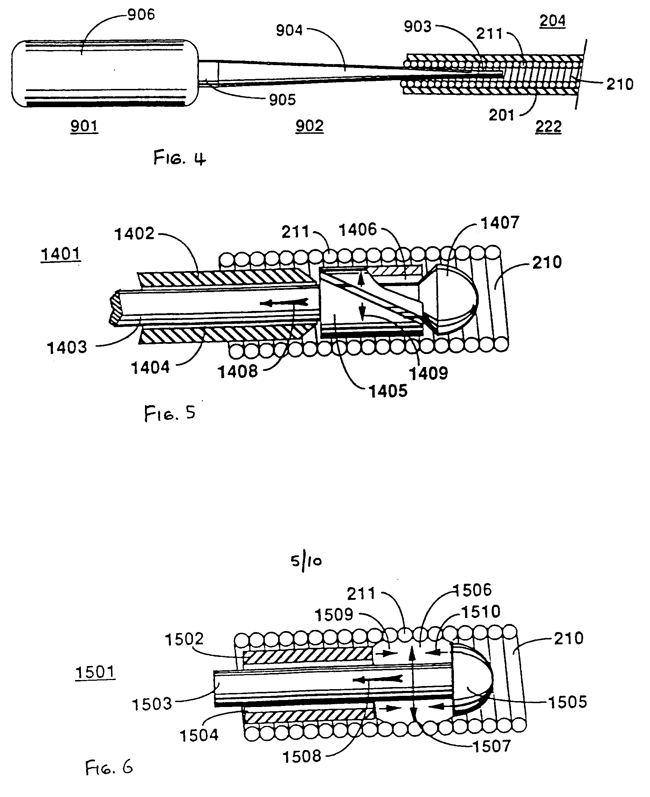

With the lockable clamp applied to the proximal end 221 of the pacemaker lead, the electrical connector (not shown) is severed from the pacemaker lead 204. The severance could deform the passageway 210 thereby presenting a false indication of the actual diameter thereof. As a consequence, the physician inserts the pointed end 903 of a tapered rod 904 of an expansion device 901 into the proximal end of hollow passageway 210 to expand coiled structure 211, as shown in FIG.4.

A physician can examine the passageway by inserting a wire guide therein and sensing for any blockages. The physician can extend the guide into the lead passageway and rotate the guide back and forth to clear any blockages caused by tissue or occluding material. The guide can also be used to determine or size the inside diameter of the lead removal apparatus that may be coaxially positioned inside coiled structure 211. When utilized as a control mechanism for stylet wire 200, the guide may also include appendages for rotating and counting the number of times the stylet wire is rotated. Having determined the lead passageway with the wire guide, several other similar guides can be individually inserted in the passageway to determine the actual inside diameter at the proximal end. The guide can also be utilized to determine if coiled structure 211 has been deformed or damaged and to determine the smallest diameter of the coiled structure and passageway.

The outer diameter of the lead removal apparatus (coil wire and stylet wire) has been selected to form a combined overall diameter which approximates the inner diameter of the longitudinal passageway of the heart lead coiled structure within a predetermined tolerance such as 0.00254 to 0.005 cms. (one or two thousandths of an inch). Stylet wire 200 is fed through the entire length of the passageway to the distal end of the coiled structure. When fully inserted into the heart lead, the distal ends of the stylet wire and coiled structure should be in close proximity. For separating the heart lead from adjacent tissue, the stylet wire may be secured anywhere along the passageway of the coiled structure past the restricting tissue. To secure the stylet wire to coiled structure 211, looped end 202 of the stylet wire is operated in a circular direction to unwind and expand wire coil 205. As a result, the turns of the wire coil and coiled structure engage and intermesh, thereby firmly securing the stylet wire to the heart lead. This prevents any extension or stretching of the heart lead and also controls and limits the movement of the lead when separator tube 212 is moved along the length of coiled structure 211 and insulating material 201 of the heart lead.

Two embodiments of the invention will now be described. They each include a control unit and a longitudinal passageway for operating the expandable part to the expanded position for securing the control unit to the elongated structure.

In the FIG.5 embodiment, the removal apparatus or stylet includes control tube 1402 and actuator rod 1403 extending through tube 1402. A diagonally-slotted sleeve 1405 is positioned between the distal ends of the control tube and actuator rod. The actuator rod also extends through hollow passageway 1406 of the sleeve. Attached to the distal end of the actuator rod is bevelled tip 1407 having an outside diameter approximating the diameter of the control tube and the nominal diameter of the slotted sleeve. Similarly, the distal end of the control tube is bevelled to engage and expand the slotted sleeve. To expand the slotted sleeve, the actuator rod is pulled, as indicated by arrow 1408, to engage the sleeve against the bevelled edges of the control tube and the rod. As a result, the sleeve is expanded to a position for engaging coiled structure 211 and securing the control tube thereto. The slotted sleeve expands in a radial direction as indicated by arrows 1409. In order to contract the sleeve in the event that withdrawal of the stylet from passageway 210 is required, the rod 1403 is pushed forward to the distal end.

In FIG.6, removal apparatus or stylet 1501 includes a control tube 1502 and extended therein an actuator rod 1503. The distal end of the actuator rod includes enlarged tip 1505 having a diameter approximating the diameter of the control tube. The device also includes expandable sleeve 1506 of pliable material which radially expands to engage 211 when rod 1503 is pulled in direction 1508. Removal of tube 1502 from passageway 210 can be achieved, if desired, by pushing forward rod 1503 and pulling tube 1502 in the direction 1508.

After the expandable part has been secured to the lead and prior to inserting separator tube 212 over the heart lead, a tie 241 of, for example, nylon cord or suture material is wrapped around proximal end 221 of the lead to secure insulating material 201 to coiled structure 211. The tie controls or limits the movement of the coiled structure within the insulating material. With the insulating material secured to the coiled structure at the proximal end, removal force is applied not only to the coiled structure, but also to the insulating material of the lead as well. This maintains the integrity of the heart lead during subsequent tissue separation from the insulating material. In those instances where the stylet wire has not been fully inserted to the distal end of the lead, the tie also prevents the coiled structure from unravelling, breaking or separating from electrode 220 or the rest of the lead.

The looped proximal end of the stylet wire can be compressed to permit separator tube 212 to be inserted thereover and over the insulating material of the heart lead. Separator tube 212 comprises a semi-rigid material, such as teflon, for sliding easily through the blood vessel and over the insulating material of the heart lead. In order to place the separator tube over the stylet, the stylet should extend at least 30.5 cms. (12 inches) beyond the person's body so that the looped end can be grasped to apply tension to the stylet. The teflon separator tube 212 is 25.4 to 30.5 cms (10 to 12 inches) long, and the stylet is typically 91.4 cms. (three feet) long.

As shown, the distal end of the teflon separator tube 212 is bevelled and includes a cutting edge or edge having a number of teeth for separating heart lead insulating material 201 from encapsulating fibrotic tissue 209. Hollow separator tube 212 has a metal bevelled tip 242 attached to the distal end thereof with, for example, a medical grade adhesive. The metal tip provides a more durable edge for separating or cutting encapsulating fibrotic tissue from the lead.

Separator tube 212 is moved and rotated along the outer surface of insulating material 201 of the heart lead to separate the lead from the blood vessel wall. After the separator tube has been moved along the entire length of the heart lead, it will abut next to the heart cavity wall as shown by phantom lines 219. The separator tube is then rotated back and forth to dislodge and separate tines 207 and the distal end of the heart lead from fibrotic tissue 218 and heart cavity wall 213. As a result, the heart lead has now been completely separated from the blood vessel and the heart cavity wall for subsequent removal. The separator tube, the stylet wire, and the heart lead are then removed from the heart cavity and surrounding blood vessel.

Should the removal of the heart lead be prevented for whatever reason in the above embodiments, the stylet can be adjusted to release the expandable part from the heart lead coiled structure.

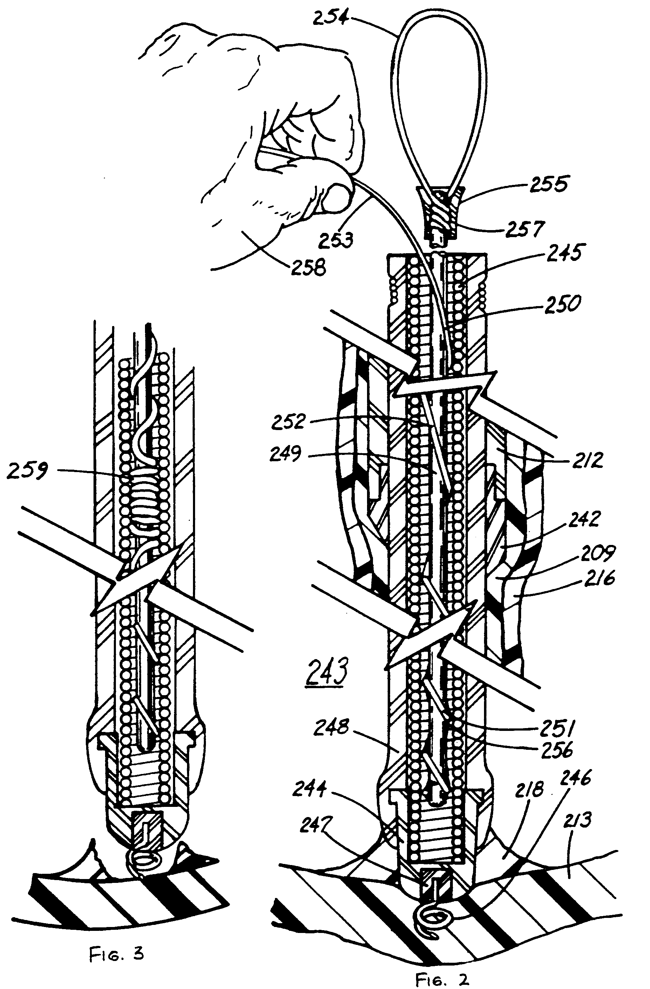

In FIG.2, electrode 244 has two cavities therein, one for receiving the coiled structure 245 and the second for receiving and securing insulated anchoring coil 246, for attaching electrode 244 to the heart tissue.

The above apparatus may be utilized for removing electrical leads or other structures from body ducts and passages as well as from body tissue that has encapsulated the lead and restricted its movement.

## Claims

1. Apparatus for removing a lead with a coiled structure (211) which lead has been implanted in biological tissue, said apparatus comprising an actuator rod (1403,1503) to be inserted into a longitudinal passageway (210) within the coiled structure via the proximal end of the latter, the rod being capable of being inserted substantially along the length of the structure; a control tube (1402,1502) with a passageway (1404,1504) through which the rod extends and is movable; and an outwardly expandable part (1405,1506) to be located within the coiled structure and for exerting lateral forces on the structure at locations remote from the proximal end of the rod when the latter is moved relative to the control tube, whereby the expandable part exerts sufficient lateral pressure on the structure to enable the latter to be removed from the biological tissue, characterised in that the expandable part is separable or separate from the control tube, and in that the control tube is longitudinally movable relative to and to engage the expandable part.

2. Apparatus according to claim 1, wherein during expansion, the distal end of the expandable part is adjacent to the distal end of the rod, and the distal end of the control tube is in engagement with the expandable part.

3. Apparatus according to claim 1 or 2, wherein the expandable part comprises two opposed surfaces which move relative to one another when longitudinal forces are exerted on the said part by the distal ends of the control tube and the actuator rod.

4. Apparatus according to claim 1,2 or 3, further comprising a second tube (212) positionable about the coiled structure for removing fibrotic tissue from the structure prior to removal thereof.

5. Apparatus according to claim 3, wherein the expandable part is a slotted sleeve (1405), with the two opposed surfaces being the edges of the slot in the sleeve.

6. Apparatus according to claim 1 or 2, wherein the expandable part is an expandable sleeve (1506) of pliable material.

7. Apparatus according to any one preceding claim. wherein the distal end of the actuator rod has an enlarged tip (1407,1505).

## Patentansprüche

1. Vorrichtung zum Entfernen einer Leitung mit einer spiralförmigen Struktur (211), wobei die Leitung in ein biologisches Gewebe implantiert wurde, wobei die Vorrichtung folgendes umfaßt: einen Betätigungsstab (1403, 1503) zum Einsetzen in einen in Längsrichtung verlaufenden Durchgang (210) in der spiralförmigen Struktur über das proximale Ende des letzteren, wobei der Stab im wesentlichen über die Länge der Struktur eingesetzt werden kann; eine Steuerröhre (1402, 1502) mit einem Durchgang (1404, 1504), durch den sich der Stab erstreckt und beweglich ist; und ein nach außen aufweitbares Teil (1405, 1506) zur Anordnung innerhalb der spiralförmigen Struktur und zum Ausüben von Querkräften auf die Struktur an von dem proximalen Ende des Stabs entfernt liegenden Stellen, wenn der Stab relativ zur Steuerröhre bewegt wird, wodurch das aufweitbare Teil einen ausreichenden Querdruck auf die Struktur ausübt, daß letztere von dem biologischen Gewebe entfernt werden kann, dadurch gekennzeichnet, daß das ausweitbare Teil von der Steuerröhre getrennt werden kann oder davon getrennt ist und daß die Steuerröhre in Längsrichtung relativ zu dem aufweitbaren Teil bewegt werden kann, um dieses in Eingriff zu nehmen.

2. Vorrichtung nach Anspruch 1, bei der sich das distale Ende des aufweitbaren Teils während der Aufweitung neben dem distalen Ende des Stabs befindet und sich das distale Ende der Steuerröhre in Eingriff mit dem aufweitbaren Teil befindet.

3. Vorrichtung nach Anspruch 1 oder 2, bei der das aufweitbare Teil zwei einander gegenüberliegende Flächen umfaßt, die sich relativ zueinander bewegen, wenn Längskräfte durch das distale Ende der Steuerröhre und des Betätigungsstabs auf das Teil ausgeübt werden.

4. Vorrichtung nach Anspruch 1, 2 oder 3, die weiterhin eine zweite Röhre (212) umfaßt, die um die spiralförmige Struktur herum positioniert werden kann, um fibrotisches Gewebe von der Struktur vor dem Entfernen der letzteren zu entfernen.

5. Vorrichtung nach Anspruch 3, bei der es sich bei dem aufweitbaren Teil um eine mit einem Schlitz versehene Hülse (1405) handelt, wobei es sich bei den beiden einander gegenüberliegenden Flächen um die Ränder des in der Hülse ausgebildeten Schlitzes handelt.

6. Vorrichtung nach Anspruch 1 oder 2, bei der es sich bei dem aufweitbaren Teil um eine aufweitbare Hülse (1506) aus biegsamem Material handelt.

7. Vorrichtung nach einem der vorhergehenden Ansprüche, bei der das distale Ende des Betätigungsstabs eine vergrößerte Spitze (1407, 1505) aufweist.

## Revendications

1. Appareil servant à retirer un conducteur comportant un boudin hélicoïdal (211) qui a été implanté dans un tissu biologique, ledit appareil comprenant une tige d'actionnement (1403, 1503) devant être insérée dans un passage longitudinal (210) à l'intérieur du boudin hélicoïdal par l'extrémité proximale de ce dernier, la tige étant à même d'être insérée dans une partie substantielle de la longueur du boudin; un tube de commande (1402, 1502) pourvu d'un passage (1404, 1504) à travers lequel la tige s'étend et peut être déplacée; et une partie expansible vers l'extérieur (1405, 1506) devant être placée à l'intérieur du boudin hélicoïdal et destinée à exercer des forces latérales sur le boudin à des endroits éloignés de l'extrémité proximale de la tige lorsque cette dernière est déplacée par rapport au tube de commande, la partie expansible exerçant une pression latérale suffisante sur le boudin pour permettre le retrait de celui-ci du tissu biologique, caractérisé en ce que la partie expansible est séparable ou séparée du tube de commande et en ce que le tube de commande peut être déplacé dans le sens longitudinal par rapport à la partie expansible de façon à venir en prise avec celle-ci.

2. Appareil suivant la revendication 1, dans lequel, au cours de l'expansion, l'extrémité distale de la partie expansible est adjacente à l'extrémité distale de la tige et l'extrémité distale du tube de commande est en prise avec la partie expansible.

3. Appareil suivant la revendication 1 ou 2, dans lequel la partie expansible comprend deux surfaces opposées qui se déplacent l'une par rapport à l'autre lorsque des forces longitudinales sont exercées sur ladite partie par les extrémités distales du tube de commande et de la tige d'actionnement.

4. Appareil suivant la revendication 1, 2 ou 3, comprenant en outre un second tube (212) pouvant être placé autour du boudin hélicoïdal pour dégager le tissu fibreux du boudin avant le retrait de celui-ci.

5. Appareil suivant la revendication 3, dans lequel la partie expansible est un manchon fendu (1405), les deux surfaces opposées formant les bords de la fente dans le manchon.

6. Appareil suivant la revendication 1 ou 2, dans lequel la partie expansible est un manchon expansible (1506) en matière souple.

7. Appareil suivant l'une quelconque des revendications précédentes, dans lequel l'extrémité distale de la tige d'actionnement a un bout de plus grand diamètre (1407, 1505).
